# EUROPÄISCHE PATENTSCHRIFT

(11) **EP 3 502 697 B1**
(45) Veröffentlichungstag und Bekanntmachung des Hinweises auf die Patenterteilung: **25.11.2020**
(21) Anmeldenummer: 17210338.4
(22) Anmeldetag: 22.12.2017
(51) Int. Cl.: G01N 33/569, G01N 33/92

(54) **NACHWEIS VON CHOLESTEROL-BINDENDEN TOXINEN**
DETECTION OF CHOLESTEROL-BINDING TOXINS
DÉTECTION DE TOXINES DE LIAISON AU CHOLESTÉROL

(43) Veröffentlichungstag der Anmeldung: 26.06.2019
(73) Patentinhaber: Justus-Liebig-Universität Gießen, 35390 Gießen (DE)
(72) Erfinder: Pillich, Helena, 35396 Gießen (DE); Chakraborty, Trinad, 35394 Giessen (DE); Hudel, Martina, 35799 Merenberg (DE); La Pietra, Luigi, 60320 Frankfurt am Main (IT)
(74) Vertreter: Stumpf, Peter

(56) Entgegenhaltungen:
- THOMAS JACOBS ET AL: "Listeriolysin O: cholesterol inhibits cytolysis but not binding to cellular membranes", MOLECULAR MICROBIOLOGY., Bd. 28, Nr. 6, 1. Juni 1998 (1998-06-01), Seiten 1081-1089, XP055466117, GB ISSN: 0950-382X, DOI: 10.1046/j.1365-2958.1998.00858.x
- CHURCHILL R L T ET AL: "Detection of Listeria monocytogenes and the toxin listeriolysin O in food", JOURNAL OF MICROBIOLOGICAL METHODS, ELSEVIER, AMSTERDAM, NL, Bd. 64, Nr. 2, 1. Februar 2006 (2006-02-01), Seiten 141-170, XP027926827, ISSN: 0167-7012 [gefunden am 2006-02-01]

## Beschreibung

Die vorliegende Erfindung liegt im Bereich der medizinischen Analytik, sowie Lebensmittelanalytik und Hygiene. Sie betrifft ein Verfahren zum verbesserten Nachweis von Cholesterol-bindenden Toxinen (cholesterol dependent cytolysins, kurz CDC) wie z.B. Listeriolysin O (LLO, produziert von *Listeria monocytogenes*) und Pneumolysin (PLY, produziert von *Streptococcus pneumoniae*) in Lebensmitteln und biologischen Proben. Insbesondere wird die Virulenzeigenschaft dieser Pathogene nachgewiesen.

CDCs werden hauptsächlich von Gram-positiven Bakterien der Gattung *Arcanobacterium, Bacillus, Clostridium, Gardnerella, Lactobacillus, Listeria* und *Streptococcus* produziert. Damit lösen einige dieser Bakterien schwere lebensbedrohliche endogene und exogene Infektionen bei Mensch und Tier aus. Dazu gehört zum einen *Streptococcus pneumoniae,* ein Besiedler des Nasopharynx und ein Erreger der Pneumonie, Sepsis und Meningitis. Jährlich sterben 1,6 Millionen Menschen, davon fast 1 Million Kinder, weltweit an einer *Streptococcus pneumoniae*-Infektion. Dieses Pathogen wird durch Tröpfcheninfektion von Mensch zu Mensch weitergegeben. Ein bedeutendes Pathogen bei Tieren ist *Streptococcus suis.* Die *Streptococcus suis*-Infektion führt beim Schwein zu Meningitis, Septikämie, Arthritis und Bronchopneumonie. Zudem gibt es unter den CDC-produzierenden Bakterien auch Pathogene, mit denen man sich durch kontaminierte Lebensmittel infizieren kann. Dazu gehören z.B. *Bacillus cereus, Clostridium botulinum* und *Listeria monocytogenes.* Die Infektion mit dem Lebensmittel- und Umweltkeim *Listeria monocytogenes* hat eine Letalitätsrate von bis zu 30%. Da das Krankheitsbild relativ unspezifisch und die Inkubationszeit sehr lange ist (bis zu 70 Tage) wird die Infektion mit *Listeria monocytogenes* oft zu spät erkannt. Dadurch werden die kontaminierten Lebensmittel nicht schnell genug entdeckt und nicht vom Markt genommen.

Cholesterol-bindende Toxine gehören zu den porenbildenden Toxinen (englisch pore forming toxin, PFT). Diese binden die Bakterien an Cholesterin in den Zellmembranen von Wirtszellen (von Mensch oder Tier) und bilden Poren von bis zu 250 Å (25 nm) Durchmesser, sodass die Zellmembranen perforiert werden. Als Folge läuft das Zytosol aus und es kommt zum Absterben der betroffenen Zellen. Die CDC-Toxine haben somit eine toxische Wirkung auf die Wirtszellen bei Mensch und Tier. Die meisten porenbildenden Toxine werden zu den Virulenzfaktoren gezählt.

Eine frühzeitige, rasche und sichere Erkennung von Cholesterol-bindenden Toxinen in biologischen Proben insbesondere in Lebensmittelproben ist daher wünschenswert, sodass kontaminierte Proben und Lebensmittel sicher identifiziert und rasch vom Markt genommen oder Patienten angemessen therapiert werden können. Eine biologische Probe umfasst im Folgenden Vollblut, Serum, Plasma, Seminalplasma, Knochenmark, Knochenmarkspunktat, Spinalflüssigkeit, peritoneale Flüssigkeit, Speichel, Tränenflüssigkeit, Gewebe, Stammzellpräparate, Urin, Stuhl, Biopsie oder Abstrich einer Körperflüssigkeit eines Menschen oder Tieres sowie eine Lebensmittelprobe oder eine Umweltprobe in Form einer Boden- und Wasserprobe. Die Probe kann dabei fest oder flüssig sein.

### Stand der Technik

Die Detektion von Cholesterol-bindenden Toxinen erfolgt derzeit hauptsächlich über den Nachweis der CDC-produzierenden Bakterien. Dazu sind verschiedene Methoden, wie das Ausplattieren auf Agarplatten bekannt. Hierbei werden die Bakterien anhand der Parameter Hämolyseverhalten, Zuckerverwertung, Kolonieform und - farbe unterschieden. Weiterhin können die Bakterien durch Antikörper-basierte (ELISA) oder Nukleinsäure-basierte (Polymerase-Ketten-Reaktion, PCR) Nachweisverfahren detektiert und unterschieden werden. Allerdings ist dabei der Nachweis von geringen Mengen an Bakterien ein Problem, sodass viele kontaminierte Proben nicht oder erst zu spät detektiert werden können.

Die Kultivierung der Bakterien auf Agarplatten erfordert eine Inkubationszeit von mind. 24 Stunden, bis zwischen pathogenen und nicht pathogenen Stämmen unterschieden werden kann. Der direkte Nachweis der Cholesterol-bindenden Toxine ist in der Diagnostik insbesondere in der Routinediagnostik nicht bekannt und nicht etabliert. Der Nachweis der Cholesterol-bindenden Toxine in biologischen Proben wie z.B. verarbeitete Lebensmittel oder Milch ist schwierig und problematisch.

Der ELISA (Enzyme-linked Immunosorbent Assay) ist ein etabliertes antikörperbasiertes immunologisches Nachweisverfahren (ein Assay), in dem Antigene durch eine enzymatische Farbreaktion nachgewiesen werden. Es gibt verschiedene Ausführungsformen des ELISAs. Bei einer Form wird das nachzuweisende Antigen direkt oder über einen Erstantikörper z.B. an eine Mikrotiterplatte gebunden und angereichert. Ein Enzym-gekoppelter Zweitantikörper führt zur Reaktion eines Farbstoffsubstrates, sobald er den Erstantikörper gebunden hat. Bei Zugabe des geeigneten Substrates bewirkt der Enzym-gekoppelte Zweitantikörper die Farbreaktion oder Chemilumineszenz, die über ein Photometer ausgewertet werden kann. So ist eine quantitative Messung der Antigenkonzentration möglich. Üblicherweise werden als Enzym Meerrettichperoxidase (HRP, horseradish peroxidase), Alkalische Phosphatase (AP) oder Glucose-Oxidase (GOP) verwendet. Der Fachmann kennt die jeweiligen Substrate, wie z.B. p-Nitrophenylphosphat (pNPP) und o-Phenyldiamin (oPD). Als Antigene werden dabei z.B. Proteine, Antikörper, Hormone oder Toxine aus einer biologischen Probe eingesetzt.

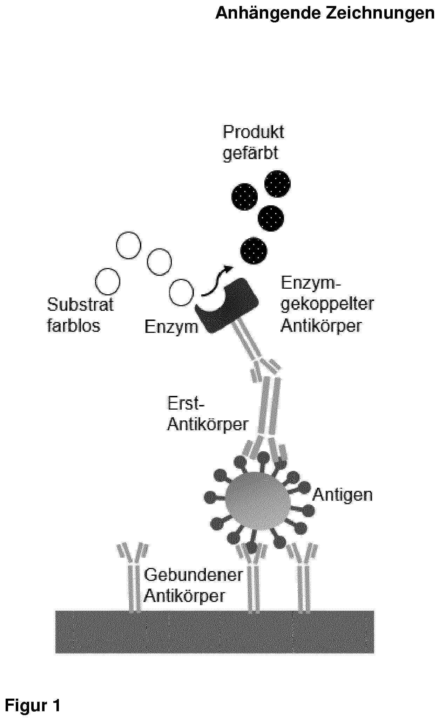

Figur 1 zeigt die beispielhaft die generelle Funktionsweise von einem indirekten Sandwich-ELISA. Die Schrift Thomas Jakobs et al., Molecular Microbiology Bd. 28, Nr. 6, 1. Juni 1998 (Seiten 1081-1089) "Listeriolysin O: cholesterol inhibits cytolysis but not binding to cellular membranes" offenbart einen Nachweis von Listeriolysin O mittels Inkubation mit Phosphatidylcholin(-Vesikeln) und anschließendem Immunoblot. Die Schrift Churchill Robin et al., Journal of Microbiological Methods, Elsevier Amsterdam Bd. 64, Nr.2, 1.Februar 2006 (Seiten 141-170) "Detection of Listeria monocytogenes and the toxin listeriolysin O in food" offenbart den Nachweis von LLO durch Blutagar oder Haemolyse-Assay.

### Aufgabe

Aufgabe der vorliegenden Erfindung ist es, ein zuverlässiges, sicheres und schnelles Nachweisverfahren von CDC-bindenden Toxinen wie z.B. LLO oder PLY in einer biologischen Probe bereitzustellen, mit dem auch sehr geringe Mengen an CDCs nachgewiesen werden können.

### Lösung der Aufgabe

Diese Aufgabe wird erfindungsgemäß gelöst durch das Nachweisverfahren von Cholesterol-bindenden Toxinen wie z.B. LLO oder PLY in einer biologischen Probe gemäß Anspruch 1. Weitere vorteilhafte Ausgestaltungen und Alternativen sind den Unteransprüchen zu entnehmen.

Überraschenderweise wurde gefunden, dass die Cholesterol-bindenden Toxine nicht nur an Cholesterol sondern darüber hinaus auch an Heparin oder an Phosphatidylcholin binden. Diese Erkenntnis wird nun vorteilhaft und erfindungsgemäß zum Nachweis der Cholesterol-bindenden Toxine eingesetzt, indem das erfindungsgemäße Verfahren insbesondere einen Anreicherungsschritt umfasst, in dem die Cholesterol-bindenden Toxine aus der biologischen Probe an Heparin oder an Phosphatidylcholin binden und somit von anderen Bestandteilen der biologischen Probe getrennt und aufkonzentriert werden. Sie können so wesentlich leichter und spezifischer isoliert werden. Zudem wird die hämolytische Aktivität der CDCs durch Heparin und Phosphatidylcholin gesteigert. Im Gegensatz dazu verlieren CDCs ihre hämolytische Aktivität, wenn sie an Cholesterol gebunden sind. Damit kann man mit Cholesterol-gebundenen CDCs keine weiteren Tests durchführen.

Durch die Aufkonzentrierung ist es möglich, den Nachweis auf CDCs selbst mit sehr geringen Konzentrationen oder Spuren an CDC erfolgreich durchzuführen. Durch diesen Anreicherungsschritt wird die Nachweisgrenze und Spezifität für die Cholesterol-bindenden Toxine erheblich gesenkt, so dass sie auch in sehr geringen Konzentrationen mit etablierten Methoden spezifisch nachgewiesen werden können.

Der Nachweis von Cholesterol-bindenden Toxinen in einer biologischen Probe erfolgt in einem immunologischen Verfahren z.B. durch ELISA oder Westernblot. Zusätzlich wird ein einfach zu verwendender Lateral Flow Test, ein sogenannter Schnelltest bereitgestellt, so dass die Testung von entnommenem biologischem Probenmaterial direkt erfolgen kann. Dieser Test kommt ohne aufwendige Labor- und Geräteausstattung aus und kann auch von Laien durchgeführt werden.

Eine biologische Probe umfasst im Folgenden Vollblut, Serum, Plasma, Seminalplasma, Knochenmark, Knochenmarkspunktat, Spinalflüssigkeit, peritoneale Flüssigkeit, Speichel, Tränenflüssigkeit, Gewebe, Stammzellpräparate, Urin, Stuhl, Biopsie oder Abstrich einer Körperflüssigkeit eines Menschen oder Tieres sowie eine Lebensmittelprobe oder eine Umweltprobe in Form einer Boden- und Wasserprobe. Die Probe kann dabei fest oder flüssig sein.

### Ausführungsbeispiele

Eigene wissenschaftliche Experimente zeigen, dass Cholesterol-bindende Toxine überraschenderweise nicht nur an Cholesterol, sondern auch an Heparin und Phosphatidylcholin binden.

Diese Erkenntnis wird erfinderisch zum qualitativen und quantitativen Nachweis der Cholesterol-bindenden Toxine in einer biologischen Probe eingesetzt. Dies erfolgt einerseits mit immunologischen Verfahren wie z.B. ELISA-Technologie, indem Heparin oder Phosphatidylcholin z.B. an die Mikrotiterplatte gebunden werden. Zusätzlich wurde überraschenderweise festgestellt, dass die Cholesterol-bindenden Toxine insbesondere an Heparin binden, das an Heparinsäulen gebunden ist. Somit ist es erstmals möglich, Cholesterol-bindende Toxine mittels FPLC (fast protein liquid chromatography) nachzuweisen und zu isolieren. Eine weitere Möglichkeit ist es, die Cholesterol-bindenden Toxine mittels Heparin-Sepharose beads aufzukonzentrieren und sie dann im Western blot nachzuweisen. Beispielhaft erfolgt der qualitative und quantitative Nachweis für Cholesterol-bindende Toxine am Beispiel von LLO (produziert von *Listeria monocytogenes*) und PLY (produziert von *Streptococcus pneumoniae*). Die Ergebnisse sind aber anzuwenden auch auf andere CDC-Toxine, die z.B. gebildet werden von *Clostridium perfringens, Bacillus cereus, Bacillus anthracis, Streptococcus pyogenes* und *Clostridium tetani.* Beispielhaft wird der Nachweis aus einer flüssigen biologischen Probe geführt. Die Ergebnisse sind aber ebenfalls anzuwenden auf feste biologische Proben.

### 1. Durchführung eines Heparin-ELISAs

Um zu beweisen, dass man generell CDCs spezifisch mittels eines Heparin-ELISAs detektieren kann, werden reine CDCs LLO und PLY eingesetzt. Dazu werden ELISA-Mikrotiterplatten mit Heparin (Santa Cruz Biotechnology) über Nacht in Coating-Puffer (z.B. 0,1 M NaHCO₃, pH 9,6) bei 4°C inkubiert. Die Mikrotiterplatten werden dreimal mit Waschpuffer (z.B. TBST: Trispuffer + 0,1% Tween-20) gewaschen und eine Stunde in Blockierpuffer (z.B. 5% Milch in TBST) bei Raumtemperatur inkubiert. Anschließend wird die Mikrotiterplatte dreimal mit Waschpuffer gewaschen und mit 0,1 µg isoliertem bzw. aufgereinigtem PLY oder LLO eine Stunde bei Raumtemperatur inkubiert. Das ungebundene Antigen wird durch dreimalige Waschschritte eliminiert und anschließend mit einem anti-PLY Antikörper (Bio-Porto, 1:1000 in TBST) oder anti-LLO Antikörper (Abcam, 1:3000 in TBST) bei Raumtemperatur inkubiert. Nach der einstündigen Inkubationszeit wird die Mikrotiterplatte dreimal mit Waschpuffer gewaschen und mit alkalischer Phosphatase-gekoppelten anti-mouse-Antikörper (Jackson ImmunoResearch Laboratories, 1:1000 in TBST) oder anti-rabbit Antikörper (Jackson ImmunoResearch Laboratories, 1:1000 in TBST) eine Stunde bei Raumtemperatur inkubiert. Nach dreimaligem Waschen mit Waschpuffer wird 100 µl des Phosphatase Substrats (Sigma-Aldrich, 1 Tablette gelöst in 10 ml Entwicklungspuffer [9,7% Diethanolamin, 0,1 mg/ml MgCl2x6H2O, pH 9,8]) gegeben und bei Raumtemperatur inkubiert. Die Reaktion wird bei 405 nm im Plattenphotometer gemessen.

Um zu zeigen, dass mittels dieses Heparin-ELISA-Tests CDCs in biologischen Proben nachweisen können, wird anstelle der reinen CDCs der Liquor cerebrospinalis von Patienten, bei denen eine Meningitis mit *Streptococcus pneumoniae* nachgewiesen wurde, verwendet. Zusätzlich werden die Inkubationsschritte verkürzt, um ein Testergebnis schneller zu erhalten. Dabei wird die Blockierzeit mit Milch, Inkubation mit dem Liquor cerebrospinalis, Inkubation mit dem anti-PLY Antikörper und dem anti-mouse Antikörper auf jeweils 15 Minuten verkürzt. Außerdem erfolgt die Inkubation mit dem Liquor cerebrospinalis bei 37°C.

Dieser Test ist ebenfalls durchführbar mit anderen flüssigen biologischen Proben. Beim Verwenden von festen biologischen Proben muss die Probe erst in einer geeigneten Flüssigkeit verflüssigt und aufgenommen werden.

Bei der Durchführung des Tests muss immer eine positive Kontrolle geführt werden. Dies erfolgt durch das Verwenden eines reinen Toxins.

Bei Verwenden des gereinigten Toxins wurde festgestellt, dass die Inkubationszeiten auf jeweils 5 Minuten herabgesetzt werden kann, wenn sie bei 37°C durchgeführt werden.

Die Nachweisgrenze liegt bei 1 µg/ml LLO.

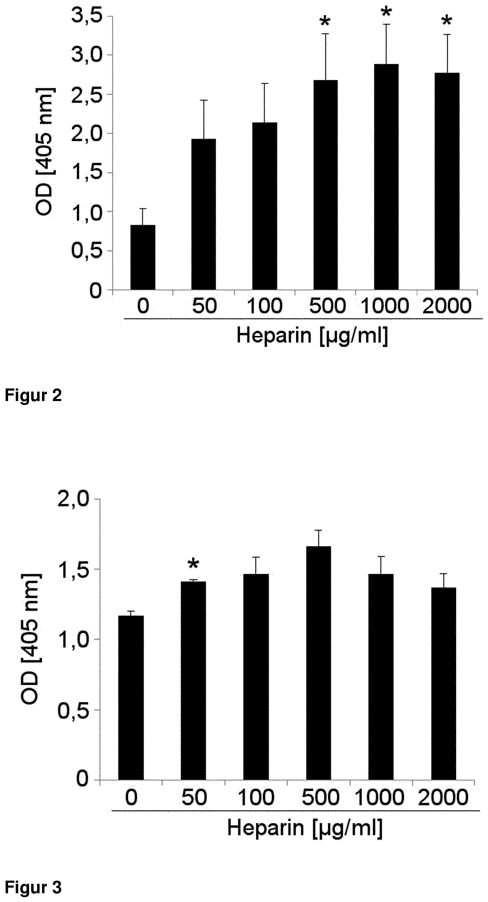

Figur 2 zeigt die Auswertung der ELISA-Platten, die mit verschiedenen Heparinkonzentrationen gecoated und mit 0,1 µg aufgereinigtem LLO inkubiert wurden. Gezeigt ist der Mittelwert aus drei unabhängigen Experimenten ± SEM. Die Sterne zeigen die durch T-Test bestimmte statistische Signifikant bezogen auf nicht mit Heparin gecoateten wells (*p<0,05).

Figur 3 zeigt die Auswertung der ELISA-Platten, die mit verschiedenen Heparinkonzentrationen gecoated wurden und mit 0,1 µg aufgereinigtem PLY inkubiert wurden. Gezeigt ist der Mittelwert aus drei unabhängigen Experimenten ± SEM. Die Sterne zeigen die durch T-Test bestimmte statistische Signifikant bezogen auf nicht mit Heparin gecoateten wells (*p<0,05).

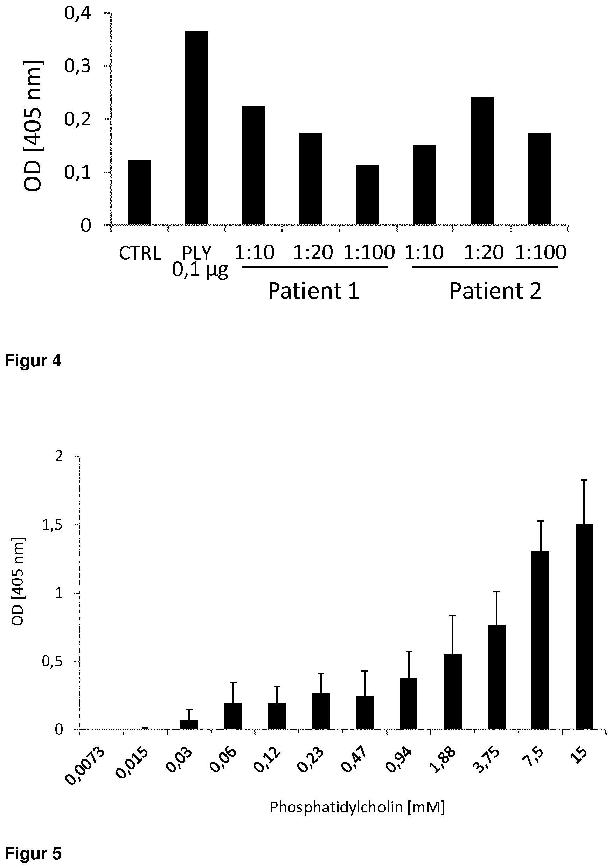

Figur 4 zeigt die Auswertung der ELISA-Platten, die mit Heparin gecoatet wurden und mit verschiedenen Konzentrationen Liquor cerebrospinalis von Patienten, bei denen eine Meningitis mit *Streptococcus pneumoniae* nachgewiesen wurde, inkubiert wurden.

### 2. Durchführung eines Phosphatidylcholin-ELISAs

ELISA-Platten werden mit Phosphatidylcholin (Sigma-Aldrich) über Nacht bei Raumtemperatur im Dunkeln gecoatet. Die ELISA-Platte wird dreimal mit TBS Puffer (Tris-buffered saline) gewaschen und mit 10 mg/ml BSA (Bovines Serumalbumin) in TBS Puffer eine Stunde bei Raumtemperatur abgeblockt. Die Blockierlösung wird abgenommen und 0,1 µg des isolierten bzw. aufgereinigten LLOs oder PLYs (gelöst in 10 mg/ml BSA-TBS) wird in die Platte gegeben.

Nach einer Stunde Inkubation bei Raumtemperatur wird die Platte dreimal mit TBS Puffer gewaschen und die Inkubation mit anti-LLO oder anti-PLY Antikörper (verdünnt in BSA-TBS), Sekundärantikörper (verdünnt in TBS) und Substrat wie oben beschrieben durchgeführt.

Dieser Test ist ebenfalls durchführbar mit flüssigen biologischen Proben. Beim Verwenden von festen biologischen Proben muss die Probe erst in einer geeigneten Flüssigkeit verflüssigt und aufgenommen werden. Um eine feste biologische Probe zu verflüssigen, wird die Probe in sterile Lösung gegeben (z.B. NaCl) und mittels Homogenisierung zerkleinert (z.B. mit FastPrep® Homogenizer). Bei der Durchführung des Tests sollte zum Vergleich eine positive Kontrolle geführt werden. Dies erfolgt durch das Verwenden des reinen Toxins.

Die Nachweisgrenze liegt bei 0,01 µg/ml LLO.

Figur 5 zeigt die Auswertung der ELISA-Platten, die mit verschiedenen Phosphatidylcholinkonzentrationen gecoated und mit 0,1 µg aufgereinigtem LLO inkubiert wurden. Gezeigt ist der Mittelwert aus drei unabhängigen Experimenten ± SEM.

Figur 6 zeigt die Auswertung der ELISA-Platten, die mit verschiedenen Phosphatidylcholinkonzentrationen gecoated und mit 0,1 µg aufgereinigtem PLY inkubiert wurden. Gezeigt ist der Mittelwert aus zwei unabhängigen Experimenten ± SEM.

### 3. Isolierung der CDCs (LLO, PLY) mittels Heparinsäulen

*L. monocytogenes* wird auf Brain Heart Infusion Broth (BHI) Agarplatten kultiviert und in eine Flüssigkultur überführt, indem 10 ml BHI Flüssigkultur in einem 100 ml Kolben mit einer Kolonie beimpft werden. Die Flüssigkultur wird bei 37°C und 180 rpm in einem Schüttelinkubator über Nacht inkubiert. Am nächsten Tag wird zwei Liter Minimalmedium mit 10 ml der Über-Nacht-Kultur beimpft und bei 37°C und 100 rpm 20-24 h inkubiert. Die Kultur wird abzentrifugiert (30 min, 9000 rpm) und der Überstand steril filtriert. Der sterile bakterielle Überstand wird mittels Cross Flow auf ca. 30 ml konzentriert.

Die Tangentialflussfiltration, auch Cross-Flow-, Tangential-Flow- oder Querstromfiltration genannt, ist eine Methode zum Filtrieren von Flüssigkeiten. Der Puffer des Konzentrats wird mit 10 mM Na-PP (pH 7,0) ausgetauscht. Das erhaltene Konzentrat wird über eine HiTrap Heparin Säule 5 ml (GE Healthcare) mittels FPLC z.B. des Äktaexplorers 100 aufgetrennt. Die Bindung der Proteine erfolgt in Gegenwart von 10 mM Na-PP (pH 7,0) und die Elution mit einem linearen Gradienten (0% bis 100%) von 10 mM Na-PP (pH 7,0) + 2 M NaCl in 20 Säulenvolumen. Der Fluss beträgt 4 ml/min.

### Minimalmedium:

16,42 g/L Na2HPO4 (autoklaviert)
6,56 g/L KH2PO4 (autoklaviert)
0,41 g/L MgSO4 (Heptahydrat) (autoklaviert)
10 g/L D-Glucose (sterilfiltriert 0,22 µm)
0,1 g/L L-Leucin (sterilfiltriert 0,22 µm)
0,1 g/L L-Isoleucin (sterilfiltriert 0,22 µm)
0,1 g/L L-Arginin (sterilfiltriert 0,22 µm)
0,1 g/L L-Methionin (sterilfiltriert 0,22 µm)
0,1 g/L L-Valin (sterilfiltriert 0,22 µm)
0,1 g/L L- Cystein (sterilfiltriert 0,22 µm)
0,6 g/L L-Glutamin (sterilfiltriert 0,22 µm)
88 mg/L Eisen(III)-citrat (sterilfiltriert 0,22 µm)
0,5 mg/L Riboflavin (gelöst in 1 M Ameisensäure, sterilfiltriert 0,22 µm)
0,5 mg/L Biotin (gelöst in 0,1 M NaOH, sterilfiltriert 0,22 µm)
1 mg/L Thiamin (sterilfiltriert 0,22 µm)
5 µg/L DL-6,8-Thioctic acid (gelöst in 70% Ethanol, sterilfiltriert 0,22 µm)

### Mit dieser Methode ist es möglich, CDCs wie LLO oder PLY zu isolieren.

Die isolierten CDCs können als positive Kontrolle eingesetzt werden.

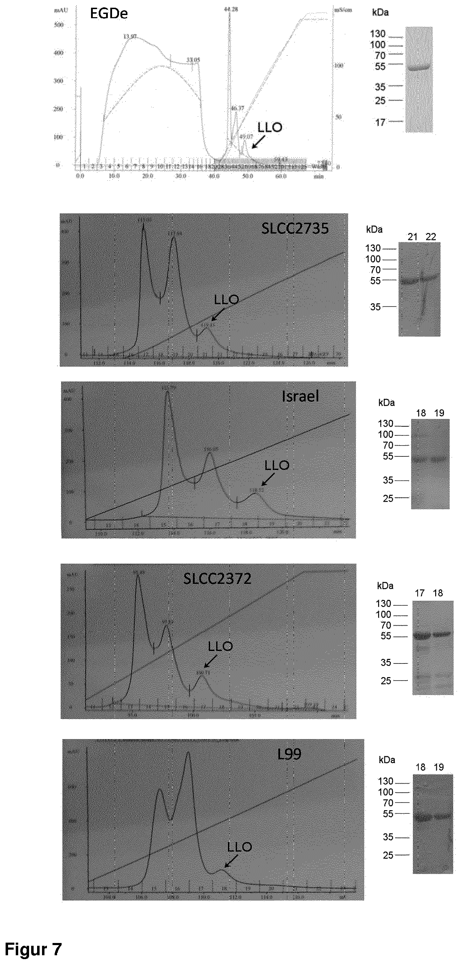

Figur 7 zeigt das FPLC-Profil von *L. monocytogenes* Kulturüberständen und das Eluat der angegebenen Fraktionen im Coomassie-Gel.

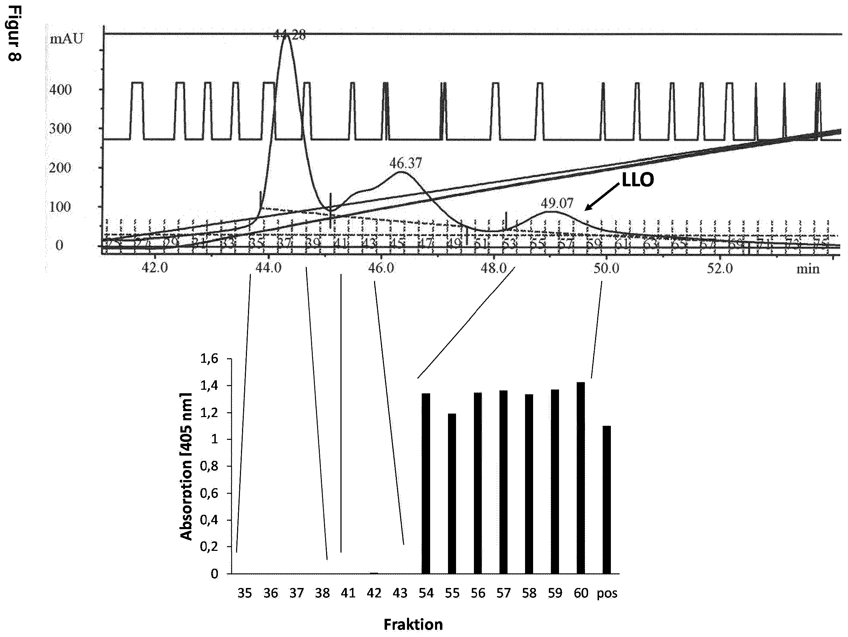

Figur 8 zeigt das FPLC-Profil von *L. monocytogenes* Kulturüberstandes und die Hämolyseeingeschaft des so isolierten LLOs bestimmt mittels des Hämolysintitertests.

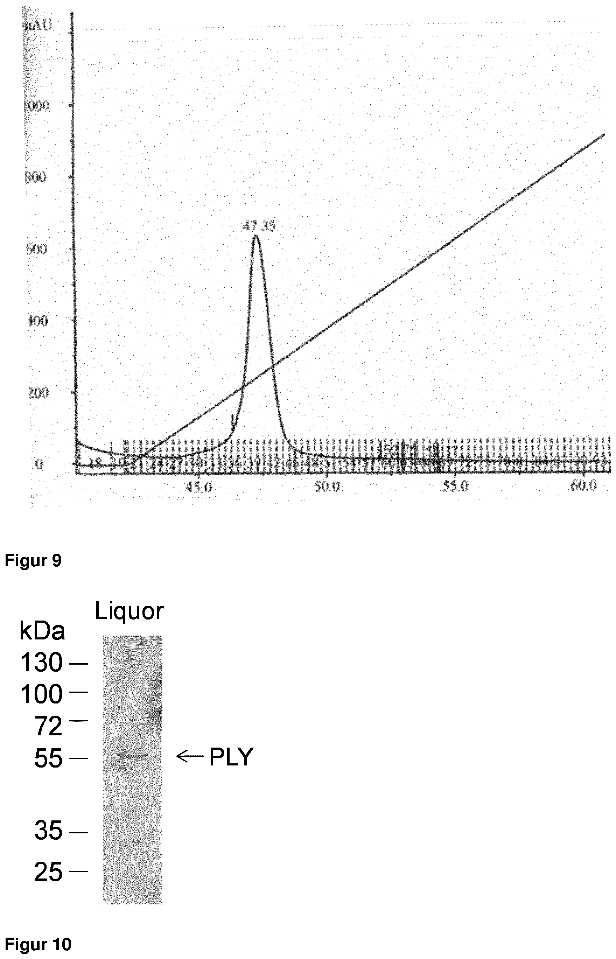

Figur 9 zeigt das FPLC-Profil vom aufgereinigtem PLY.

Mit dieser Methode werden aus 2 Liter *L. monocytogenes* Kultur 1,6 mg LLO isoliert. Alternativ werden statt dem Überstand von *Listeria monocytogenes,* Kulturüberstände von anderen CDC-produzierenden Bakterien verwendet.

### 4. Aufkonzentrierung und Nachweis von CDCs mittels Heparin-Sepharose beads

Der Liquor cerebrospinalis von Patienten, bei denen eine Meningitis mit *Streptococcus pneumoniae* nachgewiesen wurde (dies wird als positive Probe mit gesicherter Diagnose verwendet) wird über Nacht mit Heparin-Sepharose beads (Bio-Vision) bei 4°C inkubiert. Die beads werden abzentrifugiert, dreimal mit PBS gewaschen und mit 5x Auftragspuffer (62,5 mM Tris-HCI (pH 6,8), 2% (w/v) SDS, 5% (v/v) β-Mercaptoethanol, 20% (v/v) Glycerol, 0,125% (w/v) Bromphenolblau) versetzt. Die Probe wird auf ein 10%iges Trenngel aufgetragen und geblotten. PLY wird mittels der Inkubation mit anti-PLY Antikörper (Bio-Porto) und HPRgekoppelten anti-mouse Antikörper nachgewiesen (Figur 10).

Dieser Test ist ohne Abwandlung auch mit anderen flüssigen biologischen Proben durchführbar. Beim Verwenden von festen biologischen Proben muss die Probe erst verflüssigt oder in einer Flüssigkeit aufgenommen werden. Um eine feste biologische Probe zu verflüssigen, wird die Probe in sterile Lösung gegeben (z.B. NaCl) und mittels Homogenisierung zerkleinert (z.B. mit FastPrep® Homogenizer). Die Nachweisgrenze liegt bei 0,1 µg LLO.

### 5. Nachweis der CDCs mit Lateral Flow Test

Das erfindungsgemäße Nachweisverfahren von Cholesterol-bindenden Toxine wie z.B. LLO und PLY in einer biologischen Probe wird bevorzugt in einem Lateral Flow Test durchgeführt.

Der Lateral Flow Test ist ein Schnelltest, der auf einem Teststreifen ausgeführt wird. Die Durchführung des Schnelltestes ist für jedermann ohne spezielle Kenntnisse und ohne eine aufwendige Laborausstattung an jedem beliebigen Ort möglich. Das Ergebnis, ob in einer biologischen Probe Cholesterol-bindende Toxine vorhanden sind, ist einfach und schnell auswertbar.

Mit diesem Schnelltest werden die zu detektierenden Cholesterol-bindenden Toxine LLO oder PLY jeweils separat qualitativ und semi-quantitativ erfasst. Die Auswertung beinhaltet die Darstellung des Mengenverhältnisses beider Toxine zueinander in einfacher visueller Art und Weise, z.B. in einer Farbreaktion in dem mindestens einem Feld 103, die unmittelbar für das menschliche Auge sichtbar sind oder über ein Messgerät als Anzeige angezeigt werden.

Vorzugsweise weist der erfindungsgemäße Teststreifen eine Grundfläche 100 aus einem geeigneten Material, wie z.B. Nitrocellulose oder Nylon auf. Die Grundfläche ist quadratisch oder rund, vorzugsweise rechteckig. Weiterhin ist die Grundfläche mit einem geeigneten Material beschichtet, z. B. Celluloseacetat oder Cellulosenitrat, sodass sich eine Auftragsschicht 101 ergibt, die eine ebene Laufoberfläche bildet. Der Teststreifen weist mehrere voneinander isolierte Zonen auf, insbesondere eine Zone P1 zum spezifischen Nachweis von LLO ausgebildet und eine Zone P2 zum spezifischen Nachweis von PLY. Alternativ sind weitere Zonen vorhanden z.B. eine Zone K als Kontrollfeld für eine positive Kontrolle.

Zone P1 weist auf der Auftragsschicht 101 ein Auftragsfeld 102 zur Aufnahme der biologischen Probe auf. Es folgt mindestens ein Feld 103, das bereits in einem geringen Flüssigkeitsvolumen Coating-Puffer gebundenes Heparin aufweist. Alternativ sind mehrere Felder 103 vorhanden. Das Feld /die Felder 103 werden dreimal mit Waschpuffer und dann mit Blockierpuffer inkubiert, wobei alle Flüssigkeit entfernt wird. Anschließend wird zum Nachweis von PLY ein anti-PLY Antikörper bzw. zum Nachweis von LLO ein anti-LLO Antikörper aufgebracht. Wenn mehrere Felder 103 vorhanden sind, können die Tests zum Nachweis von PLY oder LLO nebeneinander angeordnet sein. Alternativ variiert die Menge an Antikörper in den Feldern 103.

Anschließend wird das Feld /die Felder 103 mit Waschpuffer gewaschen und mit alkalischer Phosphatase-gekoppelten anti-rabbit- oder anti-mouse-Antikörper (je nachdem von welchem Tier der anti-LLO Antikörper bzw. der anti-PLYAntikörper stammt) inkubiert.

Nach weiterem dreimaligem Waschen mit Waschpuffer wird das Phosphatase Substrat zugegeben.

Bei Vorhandensein von LLO bzw. PLY wird durch die enzymatische Reaktion der alkalischen Phosphatase eine deutliche Reaktion auf dem Teststreifen in Feld bzw. in den Feldern 103 sichtbar sein, die im Photometer bei 405 nm ausmessbar ist.

Alternativ ist der anti-rabbit- oder anti-mouse-Antikörper mit Goldkolloiden markiert, eine Bindung von LLO bzw. PLY führt zu einem Komplex, der durch deutliche Rotfärbung sichtbar wird und schon mit dem menschlichen Auge wahrgenommen werden kann.

Alternativ kann Zone P1 zum Nachweis von LLO verwendet werden und Zone P2 zum Nachweis von PLY.

In einer anderen Alternative erfolgt der Nachweis von LLO in Zone P1 durch Inkubation mit Heparin und in Zone P2 mit Phosphatidylcholin. In einer weiteren Alternative erfolgt der Nachweis von PLY in Zone P1 durch Inkubation mit Heparin und in Zone P2 mit Phosphatidylcholin

In einer bevorzugten Alternative des Teststreifens weist diese zusätzlich ein Feld auf, das aus einem saugenden Material, z.B. Silica-Gel besteht, so dass die Diffusion der zu testenden biologischen Probe entlang der Felder unterstützt und beschleunigt wird. Dieses Feld 104 befindet sich an dem Ende des Teststreifens, so dass Diffusion der fraglichen Probe von Feld 102 in das mindestens eine Feld 103 unterstützt und beschleunigt wird.

Der Nachweis und die Bestimmung der CDC-Konzentration in der biologischen Probe ist vorzugsweise nach 30 min abgeschlossen.

Die Nachweisgrenze liegt bei 0,01 µg/ml LLO, bzw. bei 0,01 µg/ml PLY.

### Abbildungslegenden und Bezugszeichenliste

**Figur 1** zeigt die generelle Funktionsweise eines indirekten Sandwich-ELISAs.
**Figur 2** zeigt die Auswertung der ELISA-Platten, die mit verschiedenen Heparinkonzentrationen gecoatet und mit 0,1 µg aufgereinigtem LLO inkubiert wurden. Gezeigt ist der Mittelwert aus drei unabhängigen Experimenten ± SEM. Die Sterne zeigen die durch T-Test bestimmte statistische Signifikanz bezogen auf nicht mit Heparin gecoateten wells (*p<0,05).
**Figur 3** zeigt die Auswertung der ELISA-Platten, die mit verschiedenen Heparinkonzentrationen gecoatet und mit 0,1 µg aufgereinigtem PLY inkubiert wurden. Gezeigt ist der Mittelwert aus drei unabhängigen Experimenten ± SEM. Die Sterne zeigen die durch T-Test bestimmte statistische Signifikanz bezogen auf nicht mit Heparin gecoateten wells (*p<0,05).
**Figur 4** zeigt die Auswertung der ELISA-Platten, die mit Heparin gecoatet und mit verschiedenen Konzentrationen des Patienten-Liquors *(Streptococcus pneumoni*ae-bedingte Meningitis) inkubiert wurden.
**Figur 5** zeigt die Auswertung der ELISA-Platten, die mit verschiedenen Phosphatidylcholinkonzentrationen gecoated und mit 0,1 µg aufgereinigtem LLO inkubiert wurden. Gezeigt ist der Mittelwert aus drei unabhängigen Experimenten ± SEM.

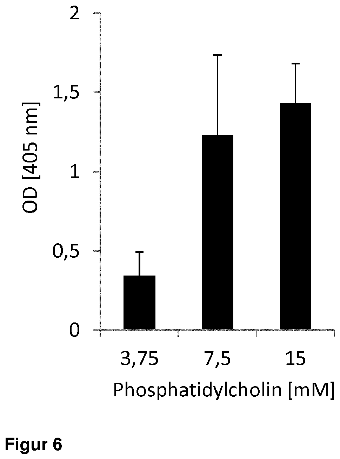

**Figur 6** zeigt die Auswertung der ELISA-Platten, die mit verschiedenen Phosphatidylcholinkonzentrationen gecoatet und mit 0,1 µg aufgereinigtem PLY inkubiert wurden. Gezeigt ist der Mittelwert aus zwei unabhängigen Experimenten ± SEM.
**Figur 7** zeigt das FPLC-Profil von *L. monocytogenes* Kulturüberständen und das Eluat der angegebenen Fraktionen im Coomassie-Gel.
**Figur 8** zeigt das FPLC-Profil von *L. monocytogenes* Kulturüberstandes und die Hämolyseeingeschaft des so isolierten LLOs bestimmt mittels des Hämolysintitertests.
**Figur 9** zeigt das FPLC-Profil von aufgereinigtem PLY.
**Figur 10** zeigt das Vorhandensein von PLY im Liquor cerebrospinalis von Patienten, bei denen eine Meningitis mit *Streptococcus pneumoniae* nachgewiesen wurde, aufkonzentriert mit Heparin-Sepharose beads und nachgewiesen mittels Westernblot.

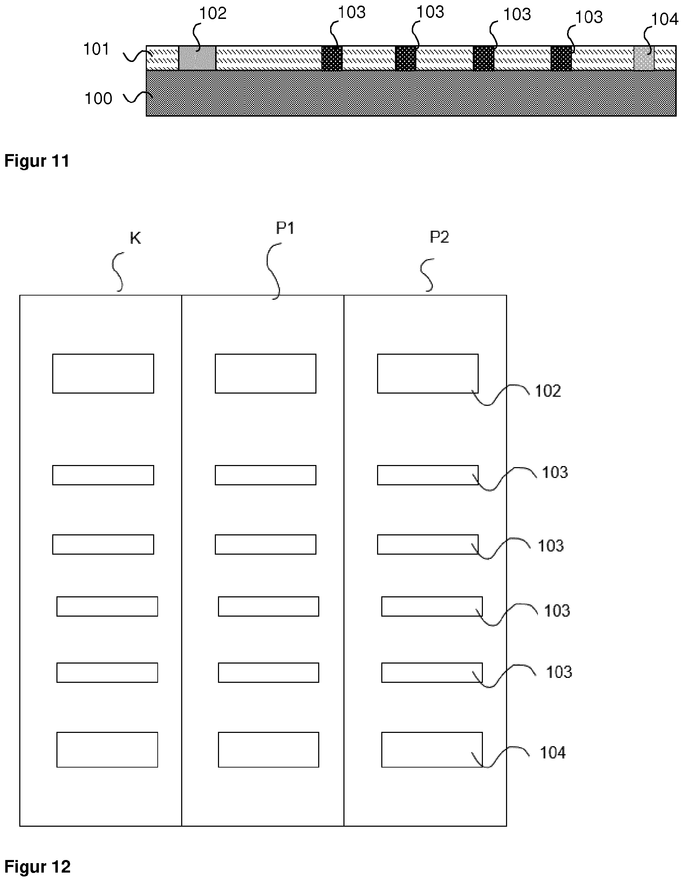

**Figur 11** zeigt den Querschnitt durch einen Lateral-Flow-Test
**Figur 12** zeigt einen exemplarischen Lateral-Flow-Teststreifen

## Patentansprüche

1. Verfahren zum Nachweis von Cholesterol-bindenden Toxinen in einer biologischen Probe **dadurch gekennzeichnet, dass** die biologische Probe mit Heparin inkubiert wird, sodass die Cholesterol-bindenden Toxine gebunden werden und mit immunologischen Nachweisverfahren nachweisbar sind.

2. Verfahren zum Nachweis von Cholesterol-bindenden Toxinen in einer biologischen Probe gemäß Anspruch 1 **dadurch gekennzeichnet, dass** die Cholesterol-bindenden Toxine Listeriolysin O (LLO) oder Pneumolysin (PLY) sind.

3. Verfahren zum Nachweis von Cholesterol-bindenden Toxinen in einer biologischen Probe gemäß Anspruch 1 oder 2 **dadurch gekennzeichnet, dass** die biologische Probe ausgewählt ist aus Vollblut, Serum, Plasma, Seminalplasma, Knochenmark, Knochenmarkspunktat, Spinalflüssigkeit, peritoneale Flüssigkeit, Speichel, Tränenflüssigkeit, Gewebe, Stammzellpräparat, Urin, Stuhl, Biopsie, Abstrich einer Körperflüssigkeit eines Menschen oder Tieres sowie eine Lebensmittelprobe oder eine Umweltprobe in Form einer Boden- oder Wasserprobe.

4. Verfahren zum Nachweis von Cholesterol-bindenden Toxinen in einer biologischen Probe gemäß einem der vorangegangenen Ansprüche 1-3 **dadurch gekennzeichnet, dass** das an Heparin gebundene Cholesterol-bindende Toxin LLO mittels spezifischer LLO-Antikörper immunologisch nachgewiesen wird.

5. Verfahren zum Nachweis von Cholesterol-bindenden Toxinen in einer biologischen Probe gemäß einem der vorangegangenen Ansprüche 1-3 **dadurch gekennzeichnet, dass** das an Heparin gebundene Cholesterol-bindende Toxin PLY mittels spezifischer PLY-Antikörper immunologisch nachgewiesen wird.

6. Verfahren zum Nachweis von Cholesterol-bindenden Toxinen in einer biologischen Probe gemäß einem der vorangegangenen Ansprüche **dadurch gekennzeichnet, dass** die biologische Probe mit Heparin-Sepharose beads inkubiert wird.

7. Verfahren zum Nachweis von Cholesterol-bindenden Toxinen in einer biologischen Probe gemäß einem der vorangegangenen Ansprüche **dadurch gekennzeichnet, dass** der immunologische Nachweis mit ELISA-Test, Westernblot oder Dot-Blot durchgeführt wird.

8. Verfahren zum Nachweis von Cholesterol-bindenden Toxinen in einer biologischen Probe gemäß einem der vorangegangenen Ansprüche **dadurch gekennzeichnet, dass** der immunologische Nachweis mit einem Lateral Flow Test nach Anspruch 11 durchgeführt wird.

9. Verfahren zum Nachweis von Cholesterol-bindenden Toxinen in einer biologischen Probe gemäß einem der vorangegangenen Ansprüche **dadurch gekennzeichnet, dass** die biologische Probe mit 50-2000 µg/ml Heparin inkubiert wird, sodass die Cholesterol-bindenden Toxine gebunden werden und mit immunologischen Nachweisverfahren nachweisbar sind.

10. Verfahren zum Nachweis von Cholesterol-bindenden Toxinen in einer biologischen Probe gemäß einem der vorangegangenen Ansprüche **dadurch gekennzeichnet, dass** die Cholesterol-bindenden Toxine in der biologische Probe in einer Konzentration ab 0,01 µg nachgewiesen werden.

11. Lateral Flow Test zur Durchführung des Verfahrens gemäß der Ansprüche 1 bis 10 **dadurch gekennzeichnet, dass** er eine Grundfläche (100) und eine Auftragsschicht (101) umfasst, wobei auf der Auftragsschicht (101) mindestens eine Zone vorhanden ist, die ein Auftragsfeld (102) zur Aufnahme einer Probe umfasst, sowie mindestens ein Feld 103, das Heparin aufweist und auf das Antikörper gegen LLO oder PLY aufgetragen werden, sodass eine Bindung von LLO oder PLY aus der biologischen Probe an die Antikörper erfolgen kann und dies zu einer Farbreaktion führt.

12. Verwendung eines Lateral Flow Tests zum Nachweis von Cholesterol-bindenden Toxinen LLO oder PLY in einer biologischen Probe gemäß einem Verfahren nach einem der Ansprüche 1-10.

## Claims

1. Method for the detection of cholesterol-binding toxins in a biological sample **characterized in that** the biological sample is incubated with heparin so that the cholesterol-binding toxins are bound and can be detected by immunological detection methods.

2. Method for the detection of cholesterol-binding toxins in a biologic sample according to claim 1, **characterized in that** the cholesterol-binding toxins are listeriolysin O (LLO) or pneumolysin (PLY).

3. Method for the detection of cholesterol-binding toxins in a biological sample according to claim 1 or 2, **characterized in that** the biological sample is selected from whole blood, serum, plasma, seminal plasma, bone marrow, bone marrow punctate, spinal fluid, peritoneal fluid, saliva, tear fluid, tissue, stem cell preparation, urine, feces, biopsy, smear of a body fluid of a human or animal and a food sample or an environmental sample in the form of a soil or water sample.

4. Method for the detection of cholesterol-binding toxins in a biologic sample according to one of the preceding claims 1-3, **characterized in that** the heparin-bound cholesterol-binding toxin LLO is detected immunologically by means of specific LLO antibodies.

5. Method for the detection of cholesterol-binding toxins in a biological sample according to one of the preceding claims 1-3, **characterized in that** the cholesterol-binding toxin PLY bound to heparin is detected immunologically by means of specific PLY antibodies.

6. Method for the detection of cholesterol-binding toxins in a biological sample according to one of the preceding claims **characterized in that** the biological sample is incubated with heparin-sepharose beads.

7. Method for the detection of cholesterol-binding toxins in a biological sample according to one of the preceding claims, **characterized in that** the immunological detection is carried out with ELISA test, Western blot or dot blot.

8. Method for the detection of cholesterol-binding toxins in a biological sample according to one of the preceding claims, **characterized in that** the immunological detection is carried out with a Lateral Flow Test according to claim 11.

9. Method for the detection of cholesterol-binding toxins in a biological sample according to one of the preceding claims, **characterized in that** the biological sample is incubated with 50-2000 µg/ml heparin so that the cholesterol-binding toxins are bound and are detectable by immunological detection methods.

10. Method for the detection of cholesterol-binding toxins in a biological sample according to one of the preceding claims, **characterized in that** the cholesterol-binding toxins are detected in the biological sample at a concentration starting from 0.01 µg.

11. Lateral flow test for carrying out the method according to claims 1 to 10, **characterized in that** it comprises a base area (100) and an application layer (101), wherein on the application layer (101) there is at least one zone comprising an application field (102) for receiving a sample, and at least one field 103 comprising heparin and to which antibodies against LLO or PLY are applied so that binding of LLO or PLY from the biological sample to the antibodies can take place and this leads to a colour reaction.

12. Use of a lateral flow assay for the detection of cholesterol-binding toxins LLO or PLY in a biological sample according to a method according to any one of Claims 1-10.

## Revendications

1. Procédé pour la détection de toxines liant le cholestérol dans un échantillon biologique, **caractérisé en ce que** l'échantillon biologique est incubé avec de l'héparine de sorte que les toxines liant le cholestérol sont liées et peuvent être détectées par des méthodes de détection immunologiques.

2. Procédé de détection de toxines fixant le cholestérol dans un échantillon biologique selon la revendication 1, **caractérisé en ce que** les toxines fixant le cholestérol sont la listeriolysine O (LLO) ou la pneumolysine (PLY).

3. Procédé pour la détection de toxines fixant le cholestérol dans un échantillon biologique selon la revendication 1 ou 2, **caractérisé en ce que** l'échantillon biologique est choisi parmi le sang entier, le sérum, le plasma, le plasma séminal, la moelle osseuse, la ponction de la moelle osseuse, le liquide céphalo-rachidien, le liquide péritonéal, la salive, le liquide lacrymal, le tissu, la préparation de cellules souches, l'urine, les selles, la biopsie, le frottis d'un fluide corporel d'un humain ou d'un animal, et un échantillon alimentaire ou un échantillon environnemental sous la forme d'un échantillon de sol ou d'eau.

4. Procédé pour la détection de toxines liant le cholestérol dans un échantillon biologique selon l'une des revendications précédentes 1-3, **caractérisé en ce que** la toxine LLO liant le cholestérol liée à l'héparine est détectée immunologiquement au moyen d'anticorps LLO spécifiques.

5. Procédé pour la détection de toxines liant le cholestérol dans un échantillon biologique selon l'une des revendications précédentes 1-3, **caractérisé en ce que** la toxine PLY liant le cholestérol liée à l'héparine est détectée immunologiquement au moyen d'anticorps PLY spécifiques.

6. Procédé pour la détection de toxines fixant le cholestérol dans un échantillon biologique selon l'une des revendications précédentes, **caractérisé en ce que** l'échantillon biologique est incubé avec des billes d'héparine-Sépharose.

7. Méthode pour la détection de toxines fixant le cholestérol dans un échantillon biologique selon l'une des revendications précédentes, **caractérisée en ce que** la détection immunologique est effectuée avec le test ELISA, le Western blot ou le dot blot.

8. Méthode pour la détection de toxines fixant le cholestérol dans un échantillon biologique selon l'une des revendications précédentes, **caractérisée en ce que** la détection immunologique est effectuée avec un test de flux latéral selon la revendication 11.

9. Procédé pour la détection de toxines liant le cholestérol dans un échantillon biologique selon l'une des revendications précédentes, **caractérisé en ce que** l'échantillon biologique est incubé avec 50-2000 µg/ml d'héparine de sorte que les toxines liant le cholestérol sont liées et sont détectables par des méthodes de détection immunologiques.

10. Procédé pour la détection de toxines liant le cholestérol dans un échantillon biologique selon l'une des revendications précédentes, **caractérisé en ce que** les toxines liant le cholestérol sont détectées dans l'échantillon biologique à une concentration de 0,01 µg ou plus.

11. Test à flux latéral pour la mise en oeuvre du procédé selon les revendications 1 à 10, **caractérisé en ce qu'**il comprend une surface de base (100) et une couche d'application (101), dans lequel sur la couche d'application (101) se trouve au moins une zone comprenant un champ d'application (102) pour recevoir un échantillon, et au moins un champ 103 qui contient de l'héparine et sur lequel sont appliqués des anticorps contre LLO ou PLY, de sorte que la liaison de LLO ou PLY de l'échantillon biologique avec les anticorps peut avoir lieu et cela conduit à une réaction colorée.

12. Utilisation d'un essai à flux latéral pour la détection de toxines liant le cholestérol LLO ou PLY dans un échantillon biologique selon une méthode conforme à l'une des revendications 1 à 10.
